# EUROPEAN PATENT SPECIFICATION

(11) **EP 3 717 026 B1**
(45) Date of publication and mention of the grant of the patent: **28.10.2026**
(21) Application number: 18821858.0
(22) Date of filing: 30.11.2018
(51) Int. Cl.: A61L 2/24, A61L 9/22, A61L 2/14, H01T 23/00, H05H 1/24

(54) **METHODS FOR STERILIZATION USING NONTHERMAL PLASMA GENERATION**
VERFAHREN ZUR STERILISATION MITTELS NICHTTHERMISCHER PLASMAERZEUGUNG
PROCÉDÉS DE STÉRILISATION PAR GÉNÉRATION D'UN PLASMA NON THERMIQUE

(30) Priority: 30.11.2017 US 201762592785 P; 29.11.2018 US 201816204567
(43) Date of publication of application: 07.10.2020
(73) Proprietor: Illinois Tool Works Inc., Glenview, IL 60025 (US)
(72) Inventor: HOGSETT, Mark Edward, Glenview Illinois 60025 (US); HEYMANN, Steven Bernard, Glenview Illinois 60025 (US); NEWBERG, Carl, Glenview Illinois 60025 (US)
(74) Representative: HGF
(86) International application number: PCT/US2018/063253
(87) International publication number: WO 2019/108898

(56) References cited:
- US-A- 4 901 194
- US-A1- 2016 051 713
- US-B2- 10 195 300
- US-B2- 8 861 166

## Description

### BACKGROUND

The present disclosure relates to sterilization and, more particularly, to systems and methods for sterilization using nonthermal plasma ionization.

Due to the historic prevalence of disease/infection transmission in medical related environments, including medical device and/or pharmaceutical manufacturing, techniques have been sought and implemented which address a reduction/elimination of transmission. Transmission reduction has been accomplished for specific enclosed tasks (e.g., autoclave replacement), but not for the general environment in which a substantial quantity of viruses, bacteria, and/or spores are being collected and redistributed (i.e., transmitted).

US2016/0051713, US4,901,194 and US8.861,166 relate to electronic ionization devices. US10195300B2 relates to a system and method for disinfecting an occupied area using germicidal radiation based on eye protection worn by persons in the area.

### SUMMARY

Methods and systems are provided for sterilization using nonthermal plasma generation (ionization), substantially as illustrated by and described in connection with at least one of the figures. The invention is defined in the appended claims.

### BRIEF DESCRIPTION OF THE DRAWINGS

These and/or other aspects will become apparent and more readily appreciated from the following description of the exemplary embodiments, taken in conjunction with the accompanying drawings.
FIG. 1 illustrates a schematic diagram of an example nonthermal plasma sterilization system, in accordance with aspects of this disclosure.
FIG. 2 is a flowchart representative of an example method to inactivate viable microorganisms using nonthermal plasma sterilization, in accordance with aspects of this disclosure.
FIG. 3 show is a flowchart representative of an example method to determine a number and arrangement of DC or AC, bipolar or steady-state ion emitter modules, in accordance with aspects of this disclosure.

The figures are not necessarily to scale. Where appropriate, similar or identical reference numbers are used to refer to similar or identical components.

### DETAILED DESCRIPTION

Disclosed example methods and systems typically address medical operating areas, laboratory, pharmaceutical or medical device manufacturing spaces comprehensively by destroying infectious organisms in place continuously both on surfaces and airborne. In particular, disclosed example methods, and systems sterilized and/or disinfect viruses, bacteria, and spores, which are attached to airborne particles, whether aerosol droplets, airborne molecular contaminants, or large particles generated by human activity in the local environment, continuously and/or in real-time with nonthermal plasma (NTP).

In some examples, an NTP-generating system is installed onto the ceiling of pre-existing structures or designed into facilities in the planning stages. Disclosed example systems are modular, reducing the difficulty retrofitting established facilities. Such disclosed systems are referred to as ceiling ionization systems, ceiling-based ionization systems, and/or room ionization systems.

Disclosed example systems include comprehensive digital NTP generation control and monitoring using computer-based software for either standalone PCs or VM (Virtual Machine) installation. In some examples, remote hand-held programming units using wireless (e.g., infrared) increase system programming and maintenance capabilities.

According to the invention, methods for inactivation of viable microorganisms include inactivating viable microorganisms in a predetermined volume by: installing a plurality of ceiling mounted direct current (DC) or alternating current (AC), bipolar or steady-state, ion emitter modules based on a geometry of the predetermined volume; and producing, using the plurality of ceiling mounted ion emitter modules, a DC or AC, bipolar or steady-state, nonthermal plasma (NTP), each of the ceiling mounted ion emitter modules comprising a high voltage power supply (HVPS).

According to the invention, the installing of the plurality of ceiling mounted ion emitter modules involves arranging the plurality of ceiling mounted ion emitter modules to have a module density in the predetermined volume based on a target ion density. In some such examples, the target ion density corresponds to a subvolume within the predetermined volume. In some examples, the installing of the plurality of ceiling mounted ion emitter modules involves providing the ceiling mounted ion emitter modules with stainless steel shrouds to protect emitters of the ceiling mounted ion emitter modules

According to the invention, methods further involve connecting the plurality of ceiling mounted ion emitter modules to a controller module and controlling the plurality of ceiling mounted ion emitter modules using the controller module. In some examples, the producing of the nonthermal plasma involves controlling at least a portion of the plurality of ceiling mounted ion emitter modules to generate the nonthermal plasma in at least one of a pulsed DC mode, a steady-state DC mode, or an AC mode.

According to methods of the invention the producing of the nonthermal plasma involves generating a nonthermal plasma having an alternating polarity. According to the invention, the producing of the nonthermal plasma involves generating the nonthermal plasma at different plasma densities using different ones of the plurality of ceiling mounted ion emitter modules. In some examples, the producing of the nonthermal plasma further involves causing the nonthermal plasma to traverse the predetermined volume.

In some example methods, the producing of the nonthermal plasma involves deactivating of bacteria, spores, fungi, and viruses present in the predetermined space in at least respective threshold fractions of the bacteria, spores, fungi, and viruses. In some examples, the producing of the nonthermal plasma involves producing a neutral net charge.

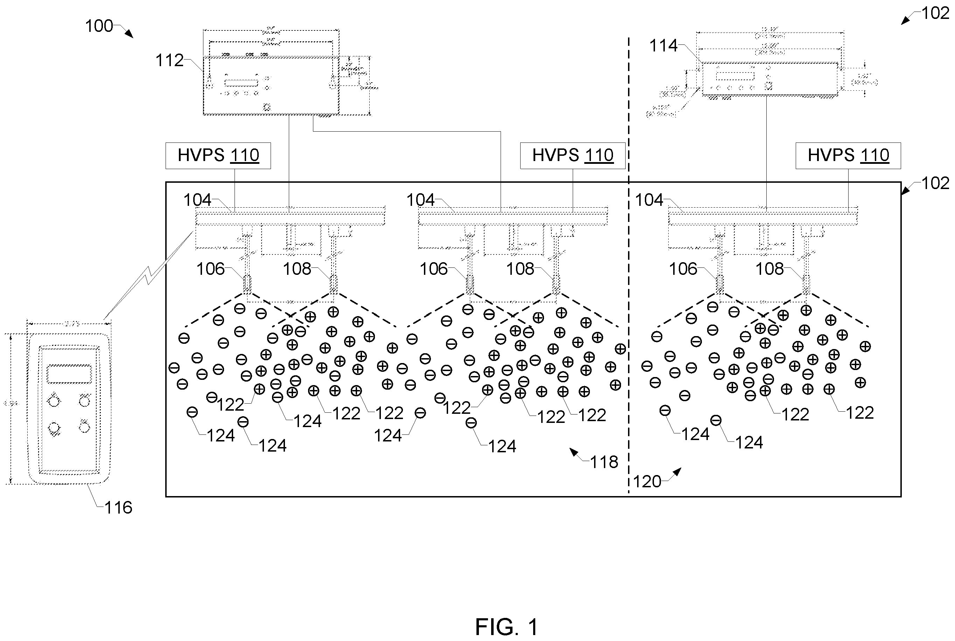

FIG. 1 illustrates a schematic diagram of an example nonthermal plasma sterilization system 100. The example system 100 of FIG. 1 may be used to provide sterilization of an entire room 102 from microorganisms such as bacteria, spores, fungi, and/or viruses, whether airborne or surface-borne. The example system 100 is modular and can be installed into a new room and/or to retrofit an existing room of any size or configuration.

The example system 100 includes one or more ceiling-mounted ion emitter modules 104, which can be of either AC or DC type and/or operate in bipolar or steady-state modes. The modules 104 may be mounted in any desired configuration, such as a spaced grid. The density and arrangement of modules 104 within a grid placement may be based on a desired level of ion coverage (e.g., a required ion density to achieve a particular sterilization level).

Each of the example ion emitter modules 104 of FIG. 1 produces a DC or AC, bipolar or steady-state, nonthermal plasma (NTP) via two separate emitters 106, 108 powered by individual DC or AC high voltage power supplies (HVPS) 110. The HVPS 110 may provide the same or different emitter outputs to each of the ion emitter modules 104. In some examples, the HVPS 110 provides voltages between 0 to 20kVDC and currents between 0 to 2µA. The example HVPS 110 may be powered with low voltage (e.g., 24VAC or VDC) wiring.

The example ion emitter modules 104, of either AC or DC type, are operated using variable pulsed intervals or steady-state and/or using varying power settings to generate a large and expanding NTP. The DC or AC, bipolar or steady-state, NTP generated by the ion emitter modules 104 expands to fill the enclosed room 102 with an alternating polarity NTP. The example ion emitter modules 104 may be operated using plasma production modes such as pulsed DC, steady-state DC, standby, and/or any combination of modes. The ion emitter modules 104 are constructed with materials exhibiting high resistance to disinfection chemical treatments commonly applied to controlled medical, laboratory, or manufacturing spaces. Example materials include stainless steel and/or any other materials defined by industry specification.

The ion emitter modules 104 (DC or AC, bipolar or steady-state) in the system 100 are digitally controlled using one or more digital controllers 112, 114. The example digital controller 112 controls a first set of the ion emitter modules 104, and the digital controller 114 controls a second set of the ion emitter modules 104. The example digital controllers 112, 114 may differ in the number of ion emitter modules 104 that can be controlled and/or the features that can be provided. The ion emitter modules 104 are individually addressable by the controllers 112, 114. In some examples, one of the controllers 112, 114 in a multiple-controller system may be designated as a master controller.

The ion emitter modules 104 may be connected in parallel and/or daisy-chained, for power and/or control purposes. The ion emitter modules 104 are individually programmable via the digital controllers 112, 114, via a software manager program executed on a general-purpose computer, and/or via a handheld programming device. The example software manager program may, for example, display emitter pod layout maps with visual and/or system alarm mode processing and/or manage emitter pod maintenance alarms, emitter pod failures, performance degradation, and/or any other configuration and/or diagnostic information. The example controllers 112, 114 and/or the ion emitter modules 104 are accessible and/or programmable via a hand-held terminal at the location of the digital controller 112, 114 or specific emitter modules.

The example ion emitter modules 104 have wireless communication ability (e.g., infrared, WiFi, Bluetooth, NFC, Zigbee, etc.) and can be programmed individually from directly below via hand-held wireless programming devices 116. The programming devices 116 may be used to verify configurations of one or more ion emitter modules 104.

The example HVPSs 110 and/or the example controllers 112, 114 may be located within the room 102, directly adjacent the room 102 (e.g., above the ceiling), and/or in a remote location (e.g., a different room) from the ion emitter modules 104.

While the example system 100 is illustrated with three ion emitter modules 104, any number of ion emitter modules 104 and/or controllers 112, 114 may be used, based on the desired ionization coverage for a particular room and/or portion of a room. The example ceiling mounted system 100 may be configured uniformly cover an entire ceiling to floor space of the room 102 in which the system 100 is installed.

In some examples, sub-volumes 118, 120 or sub-areas of a given room can be selectively ionized to provide local targeted coverage, and/or different sub-volumes or sub-areas of the room 102 can be ionized with different ion densities. For example, the sub-volume 118 of the room 102 may have a higher density of the ion emitter modules 104 than the sub-volume 120 of the room 102. The number and/or arrangement of the ion emitter modules 104 is selected and/or determined based on the ion densities of the room 102 and/or sub-volumes of the room 102.

In operation, the ion emitter modules 104 produce a DC or AC, bipolar or steady-state, nonthermal plasma including positive ions 122 and negative ions 124 to inactivate airborne and/or surface-borne microorganisms (e.g., bacteria, spores, fungi, and/or viruses) while producing a substantially neutral net charge. The ion emitter modules 104 produce a nonthermal plasma having an alternating polarity and may simultaneously generate ions having both positive and negative polarities (e.g., using different electrodes).

Based on the arrangements of the ion emitter modules 104 and/or plasma production modes, the example ion emitter modules 104 may generate the nonthermal plasma at different plasma densities for different portions of the room 102. The ion emitter modules 104 generate the nonthermal plasma to cause the ions 122, 124 to traverse the entirety of the room 102 and/or at least a predetermined volume of the room 102.

Example implementations of the ion emitter modules 104 and the controllers 112, 114, are described in U.S. Patent No. 8,861,166 to Richie, Jr., et al., and U.S. Patent No. 4,901,194, to Steinman, et al.

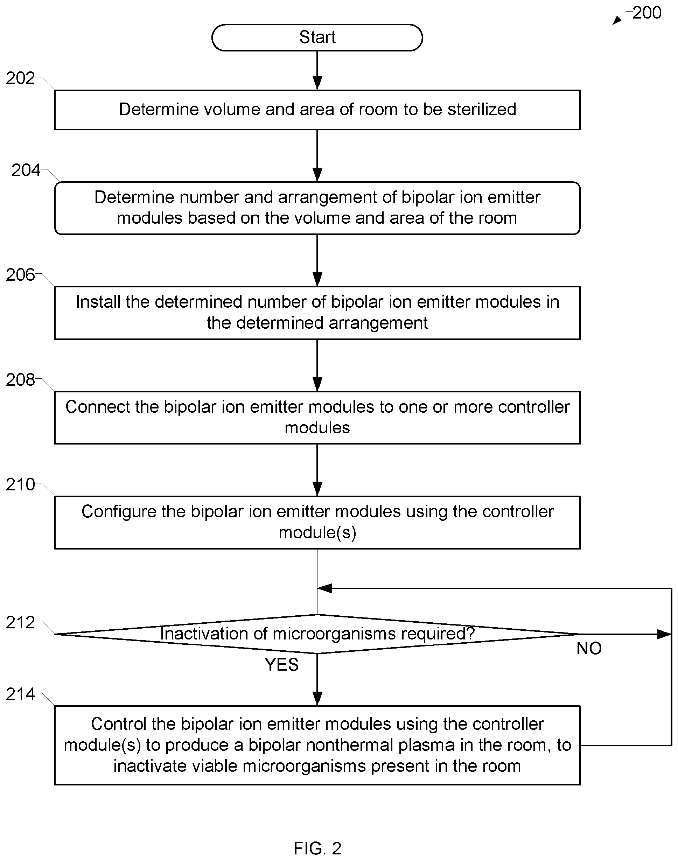

FIG. 2 is a flowchart representative of an example method 200 to inactivate viable microorganisms using nonthermal plasma sterilization. The example method 200 is described below with reference to the example system 100 of FIG. 1.

At block 202, a volume and/or area of a room that is to be sterilized (e.g., the room 102) is determined. The sterilization may be continuous or noncontinuous, and/or different portions of the room 102 (e.g., sub-volumes 118, 120) may have different sterilization requirements. In some examples, a microorganism reduction level (e.g., a log-kill ratio) may be defined for the room 102.

At block 204, the number and arrangement of ion emitter modules are determined based on the volume and area of the room 102. For example, a number of ion emitter modules 104 needed to provide one or more ionization level(s) necessary to achieve a particular sterilization, volumetric ion coverage, and/or area ion coverage, may be determined based on the volume and/or area of the room 102, height of the room 102, airflow rate(s) within the room 102, and/or ionization capacity of the ion emitter modules 104, wherein the factors for installing a plurality of ceiling mounted ion emitter modules, which are limiting the scope of protection, are those outlined in the appended claims.

At block 206, the determined number of ion emitter modules 104 are installed in the determined arrangement. For example, the ion emitter modules 104 are installed on the ceiling of the room 102 to provide the desired ionization. Installation of the ion emitter modules 104 may further involve connecting the ion emitter modules 104 to respective HVPSs 110.

At block 208, the ion emitter modules 104 are connected to one or more controller modules (e.g., the digital controllers 112, 114 of FIG. 1). For example, the ion emitter modules 104 may be coupled to the digital controllers 112, 114 via a bus, point-to-point connections, wireless connections, and/or any other connections. In some examples, a first subset of the ion emitter modules 104 are coupled to the digital controller 112, up to a control capacity of the digital controller 112. A second subset of the ion emitter modules 104 is connected to the digital controllers 114 (up to a control capacity of the digital controllers 114).

At block 210, the ion emitter modules 104 are configured using the connected controller module(s) 112, 114. For example, the ion emitter modules 104 may be configured for one or more plasma production modes (e.g., pulsed DC, steady-state DC, standby, and/or any combination of modes), a pulsing interval, and/or any other configurable aspects, at least some of which are described in U.S. Patent No. 8,861,166 and U.S. Patent No. 4,901,194.

At block 212, the example controller(s) 112, 114 may determine whether inactivation of microorganisms is required. According to the invention, the controller(s) 112, 114 more generally determine whether ionization is to be performed, which is based on a schedule and one or more sensors indicative of a sterilization level of the room 102. In some examples (not falling under the scope of protection), inactivation of microorganisms is continuous and, thus, block 212 may be omitted.

When inactivation of microorganisms is required (block 212), at block 214 the controller(s) 112, 114 control the ion emitter modules 104 to produce a nonthermal plasma in the room 102, to inactivate viable microorganisms in the room 102. For example, the ion emitter modules 104 generate the positive ions 122 and the negative ions 124 to result in a substantially neutral charge within the room 102, while the positive ions 122 and/or the negative ions 124 inactivate airborne and/or surface-borne microorganisms.

In the example method 200, blocks 212 and 214 iterate to generate nonthermal plasma as appropriate to provide sterilization to the room 102.

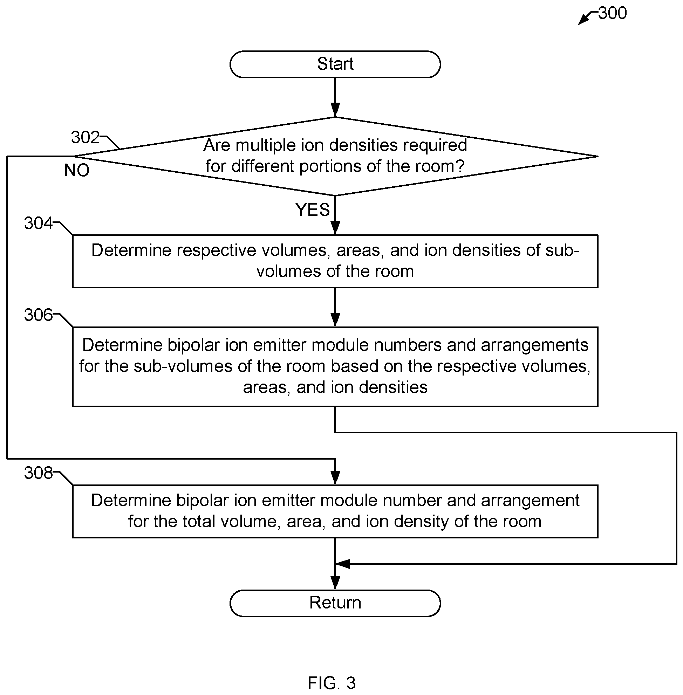

FIG. 3 show is a flowchart representative of an example method 300 to determine a number and arrangement of the ion emitter modules 104 of FIG. 1. The example method 300 may be performed to implement block 204 of FIG. 2, and may begin after performing block 202 of FIG. 2.

When multiple ion densities are required for different portions of a room (block 302), at block 304, respective volumes, areas, and ion densities of sub-volumes of the room 102 (e.g., sub-volumes 118, 120) are determined. At block 306, ion emitter module numbers and arrangements for the sub-volumes 118, 120 of the room 102 are determined based on the respective volumes, areas, and ion densities.

When multiple ion densities are not required for different portions of a room (e.g., a same ion density for the room 102 is acceptable) (block 302), at block 308 the ion emitter module number and arrangement are determined for the total volume, area, and ion density of the room 102.

After determining ion emitter module number(s) and arrangement(s), the example method 300 ends. The example method 200 may then resume with block 206.

The present methods and systems may be realized in hardware and/or a combination of i) hardware and ii) software and/or firmware. The present methods and/or systems may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may include a general-purpose computing system with a program or other code that, when being loaded and executed, controls the computing system such that it carries out the methods described herein. Another typical implementation may comprise one or more application specific integrated circuit or chips. Some implementations may comprise a non-transitory machine-readable (e.g., computer readable) medium (e.g., FLASH memory, optical disk, magnetic storage disk, or the like) having stored thereon one or more lines of code executable by a machine, thereby causing the machine to perform processes as described herein. As used herein, the term "non-transitory machine-readable medium" is defined to include all types of machine readable storage media and to exclude propagating signals.

As utilized herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or." As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

While the present method and/or system has been described with reference to certain implementations, it will be understood by those skilled in the art that various changes may be made and equivalents may be substituted without departing from the scope of the present method and/or system. In addition, many modifications may be made to adapt a particular situation or material to the teachings of the present disclosure without departing from its scope. Therefore, the present method and/or system are not limited to the particular implementations disclosed. Instead, the present method and/or system will include all implementations falling within the scope of the appended claims.

## Claims

1. A method for inactivation of viable microorganisms, the method comprising:
inactivating viable microorganisms in a predetermined volume by:
installing (206) a plurality of ceiling mounted direct current, DC, or alternating current, AC, bipolar or steady-state, ion emitter modules (104) based on a geometry of the predetermined volume, wherein the installing (206) of the plurality of ceiling mounted ion emitter modules (104) comprises arranging the plurality of ceiling mounted ion emitter modules (104) to have a module density in the predetermined volume based on a target ion density; and
producing (214), using the plurality of ceiling mounted ion emitter modules (104), a DC or AC, bipolar or steady-state, nonthermal plasma, NTP, each of the ceiling mounted ion emitter modules (104) comprising a high voltage power supply, HVPS;
connecting (208) the plurality of ceiling mounted ion emitter modules (104) to a controller module (112,114); and
controlling the plurality of ceiling mounted ion emitter modules (104) using the controller module (112,114)
wherein the controller is configured to determine whether ionization is to be performed, based on a schedule and one or more sensors indicative of a sterilization level of the room 102 and; wherein the producing (214) of the nonthermal plasma comprises generating a nonthermal plasma having an alternating polarity and comprises generating the nonthermal plasma at different plasma densities using different ones of the plurality of ceiling mounted ion emitter modules (104).

2. The method as defined in claim 1, wherein the target ion density corresponds to a subvolume within the predetermined volume.

3. The method as defined in claim 1, wherein the producing (214) of the nonthermal plasma comprises controlling at least a portion of the plurality of ceiling mounted ion emitter modules (104) to generate the nonthermal plasma in at least one of a pulsed DC mode, a steady-state DC mode, or an AC mode.

4. The method as defined in claim 1, wherein the installing (206) of the plurality of ceiling mounted ion emitter modules (104) comprises providing the ceiling mounted ion emitter modules (104) with stainless steel shrouds to protect emitters (106,108) of the ceiling mounted ion emitter modules (104).

5. The method as defined in claim 1, wherein the producing (214) of the nonthermal plasma comprises deactivating of bacteria, spores, fungi, and viruses present in the predetermined volume by a predetermined log-kill ratio defined for the predetermined volume.

6. The method as defined in claim 1, wherein the producing (214) of the nonthermal plasma comprises producing a neutral net charge.

7. The method as defined in claim 1, wherein the installing (206) of the plurality of ceiling mounted ion emitter modules (104) comprises arranging the plurality of ceiling mounted ion emitter modules (104) based on a first target ion density for a first subvolume of the predetermined volume and a second target ion density for a second subvolume of the predetermined volume, the second target ion density being different than the first target ion density.

## Patentansprüche

1. Verfahren zur Inaktivierung lebensfähiger Mikroorganismen, wobei das Verfahren Folgendes aufweist:
Inaktivieren lebensfähiger Mikroorganismen in einem vorbestimmten Volumen durch:
Installieren (206) einer Vielzahl von an der Decke angebrachten Gleichstrom- (DC) oder Wechselstrom- (AC), bipolaren oder stationären Ionenemittermodulen (104) basierend auf einer Geometrie des vorbestimmten Volumens, wobei das Installieren (206) der Vielzahl von an der Decke angebrachten Ionenemittermodulen (104) umfasst, die Vielzahl von an der Decke angebrachten Ionenemittermodulen (104) so anzuordnen, dass sie eine Moduldichte in dem vorbestimmten Volumen basierend auf einer Zielionendichte aufweisen; und
Erzeugen (214), unter Verwendung der Vielzahl von an der Decke angebrachten Ionenemittermodulen (104), eines DC- oder AC-, bipolaren oder stationären nichtthermischen Plasmas, NTP, wobei jedes der an der Decke angebrachten Ionenemittermodule (104) eine Hochspannungsstromversorgung, HVPS, aufweist;
Verbinden (208) der Vielzahl von an der Decke angebrachten Ionenemittermodulen (104) mit einem Steuermodul (112,114); und
Steuern der Vielzahl von an der Decke angebrachten Ionenemittermodulen (104) unter Verwendung des Steuermoduls (112,114)
wobei die Steuerung konfiguriert ist, um zu bestimmen, ob eine Ionisierung durchgeführt werden soll, basierend auf einem Zeitplan und einem oder mehreren Sensoren, die ein Sterilisationsniveau des Raums 102 anzeigen, und;
wobei das Erzeugen (214) des nichtthermischen Plasmas das Erzeugen eines nichtthermischen Plasmas mit einer alternierenden Polarität aufweist und das Erzeugen des nichtthermischen Plasmas bei unterschiedlichen Plasmadichten unter Verwendung unterschiedlicher der Vielzahl von an der Decke angebrachten Ionenemittermodulen (104) aufweist.

2. Verfahren nach Anspruch 1, wobei die Zielionendichte einem Untervolumen innerhalb des vorbestimmten Volumens entspricht.

3. Verfahren nach Anspruch 1, wobei das Erzeugen (214) des nichtthermischen Plasmas das Steuern von zumindest einem Abschnitt der Vielzahl von an der Decke angebrachten Ionenemittermodulen (104) aufweist, um das nichtthermische Plasma in zumindest einem von einem gepulsten Gleichstrommodus, einem stationären Gleichstrommodus oder einem Wechselstrommodus zu erzeugen.

4. Verfahren nach Anspruch 1, wobei das Installieren (206) der Vielzahl von an der Decke angebrachten Ionenemittermodulen (104) das Bereitstellen der an der Decke angebrachten Ionenemittermodule (104) mit Ummantelungen aus rostfreiem Stahl aufweist, um Emitter (106,108) der an der Decke angebrachten Ionenemittermodule (104) zu schützen.

5. Verfahren nach Anspruch 1, wobei das Erzeugen (214) des nichtthermischen Plasmas das Deaktivieren von in dem vorbestimmten Volumen vorhandenen Bakterien, Sporen, Pilzen und Viren mit einer vorbestimmten logarithmischen Keimreduktionsrate aufweist, die für das vorbestimmte Volumen definiert ist.

6. Verfahren nach Anspruch 1, wobei das Erzeugen (214) des nichtthermischen Plasmas das Erzeugen einer neutralen Nettoladung aufweist.

7. Verfahren nach Anspruch 1, wobei das Installieren (206) der Vielzahl von an der Decke angebrachten Ionenemittermodulen (104) das Anordnen der Vielzahl von an der Decke angebrachten Ionenemittermodulen (104) basierend auf einer ersten Zielionendichte für ein erstes Untervolumen des vorbestimmten Volumens und einer zweiten Zielionendichte für ein zweites Untervolumen des vorbestimmten Volumens aufweist, wobei die zweite Zielionendichte sich von der ersten Zielionendichte unterscheidet.

## Revendications

1. Procédé pour l'inactivation de micro-organismes viables, le procédé comprenant :
l'inactivation de micro-organismes viables dans un volume prédéterminé par :
l'installation (206) d'une pluralité de modules émetteurs d'ions montés au plafond (104), à courant continu (CC) ou courant alternatif (CA), bipolaire ou à régime permanent, sur la base d'une géométrie du volume prédéterminé, dans lequel l'installation (206) de la pluralité de modules émetteurs d'ions montés au plafond (104) comprend l'agencement de la pluralité de modules émetteurs d'ions montés au plafond (104) de façon à avoir une densité de modules dans le volume prédéterminé sur la base d'une densité ionique cible ; et
la production (214), en utilisant la pluralité de modules émetteurs d'ions montés au plafond (104), d'un plasma non thermique (NTP) CA ou CC, bipolaire ou en régime permanent, chacun des modules émetteurs d'ions montés au plafond (104) comprenant une alimentation haute tension, HVPS ;
la connexion (208) de la pluralité de modules émetteurs d'ions montés au plafond (104) à un module de commande (112, 114) ; et
la commande de la pluralité de modules émetteurs d'ions montés au plafond (104) à l'aide du module de commande (112, 114)
dans lequel le dispositif de commande est configuré pour déterminer si l'ionisation doit être réalisée, sur la base d'un calendrier et d'un ou plusieurs capteurs indicatifs d'un niveau de stérilisation de la pièce 102 et ;
dans lequel la production (214) du plasma non thermique comprend la génération d'un plasma non thermique à alternance de polarité et comprend la génération du plasma non thermique à des densités de plasma différentes en utilisant des modules différents parmi la pluralité de modules émetteurs d'ions montés au plafond (104).

2. Procédé selon la revendication 1, dans lequel la densité ionique cible correspond à un sous-volume au sein du volume prédéterminé.

3. Procédé selon la revendication 1, dans lequel la production (214) du plasma non thermique comprend la commande d'au moins une portion de la pluralité de modules émetteurs d'ions montés au plafond (104) pour générer le plasma non thermique dans au moins l'un parmi un mode CC pulsé, un mode CC en régime permanent, ou un mode CA.

4. Procédé selon la revendication 1, dans lequel l'installation (206) de la pluralité de modules émetteurs d'ions montés au plafond (104) comprend le fait de munir les modules émetteurs d'ions montés au plafond (104) de carénages en acier inoxydable pour protéger les émetteurs (106, 108) des modules émetteurs d'ions montés au plafond (104).

5. Procédé selon la revendication 1, dans lequel la production (214) du plasma non thermique comprend l'inactivation des bactéries, des spores, des champignons et des virus présents dans le volume prédéterminé par une réduction logarithmique prédéterminée définie pour le volume prédéterminé.

6. Procédé selon la revendication 1, dans lequel la production (214) du plasma non thermique comprend la production d'une charge nette neutre.

7. Procédé selon la revendication 1, dans lequel l'installation (206) de la pluralité de modules émetteurs d'ions montés au plafond (104) comprend l'agencement de la pluralité de modules émetteurs d'ions montés au plafond (104) sur la base d'une première densité ionique cible pour un premier sous-volume du volume prédéterminé et d'une deuxième densité ionique cible pour un deuxième sous-volume du volume prédéterminé, la deuxième densité ionique cible étant différente de la première densité ionique cible.
